# EUROPEAN PATENT APPLICATION

(11) **EP 4 387 273 A1**
(43) Date of publication of application: **19.06.2024**
(21) Application number: 23212533.6
(22) Date of filing: 28.11.2023
(51) Int. Cl.: H04R 25/00, H04R 1/10

(54) **FITTING SYSTEM, AND METHOD OF FITTING A HEARING DEVICE**

(30) Priority: 15.12.2022 DK PA202270616
(71) Applicant: GN Hearing A/S, 2750 Ballerup (DK)
(72) Inventor: Pedersen, Brian Dam, 2750 Ballerup (DK); Dyssegard, Christoffer, 2750 Ballerup (DK)
(74) Representative: GN Store Nord A/S

(57) **Abstract**

A method and a system for fitting a hearing device to a hearing loss of a user is devised. The method utilizes a data pool of existing hearing device fittings for training a deep neural network of the system to be capable of predicting a dataset of gain value ranges for a hearing device fitting when the system is presented with a user profile comprising an audiogram, a set of user data, and a proposed hearing device. The dataset of gain value ranges predicted by the system may be subjected to statistical methods for providing a set of gain values suitable for being applied directly to the hearing device to be fitted. The system and the method may beneficially be used for automatically fitting an OTC hearing device, for checking a proposed hearing device fitting, or for updating an existing hearing device fitting, for instance when relevant data in the user profile changes.

## Description

### FIELD

This disclosure concerns hearing devices. More specifically, hearing devices capable of being fitted to compensate for a hearing loss of a user.

### BACKGROUND

A wide range of contemporary fitting rules can be used to fit a hearing device to a patient having a hearing loss. These fitting rules may be proprietary fitting rules tied to a specific hearing device manufacturer, fitting rules tied to a particular best fitting practice, or fitting rules provided by a third-party entity. However, these third-party fitting rules cannot take proprietary hardware specifications into account, and proprietary fitting rules might not be up to date, e.g., whenever new hearing device models are introduced to the market. Additionally, the fitting rules may have been developed based on a specific group of patients that does not necessarily generalize to an entire population. As a result, fitting rules and the resulting fittings might not be as tailored to the individual and the specific hardware as desired. This generates more work for the hearing care professionals (HCPs) as they have to spend more time on fine-tuning previously fitted hearing devices and thus potentially need to schedule more appointments with the same patient.

Furthermore, it poses a problem for the recently introduced OTC (over-the-counter) hearing device category where the user is guided through the fitting procedure by an automated or semi-automated process after purchase of the hearing device without involving an HCP in the initial fitting of the hearing device to the user's hearing loss. Extra care in designing and maintaining the automated fitting procedure is therefore needed in order to ensure a good, first fit.

Machine learning algorithms are getting increasingly common for solving complex problems, especially within the field of pattern recognition and similar problems requiring nontrivial solutions. Contemporary machine learning systems are, e.g., capable of recognizing letters from a scanned page of text, interpret and act upon spoken messages, recognize a person's face from a picture, predict a weather forecast from meteorological measurements and play a range of different games on a level comparable to any skilled human player. Many complex problems may be expressed as functions, where a set of input parameters presented to a trained machine learning system yields a sensible, reproducible output within the specific context the machine learning system is trained to provide.

As discussed in the foregoing, the fitting of a hearing device to alleviate a specific hearing loss is not a trivial task. The inventors have realized that a deep neural network has inherent features that would be helpful in solving the complex task of fitting a hearing device to a given hearing loss. The chosen approach in order to accomplish this task is described in greater detail in the following.

Deep neural networks (DNN) are artificial neural networks having multiple layers between an input layer and an output layer. A DNN always comprises the same components equivalent to neurons, synapses, weights, biases, and functions. These components, considered as a whole, thus function similarly to a human brain, and can be trained like any other machine learning algorithm.

If a task can be expressed mathematically, it can, in theory, be solved by a DNN. Once the DNN has been sufficiently trained, it may be considered as a 'black box' capable of predicting proper solutions to problems resembling the problems used for training. Many training strategies for training DNNs exist, dependent on the size and accuracy of the applied training set, the tolerance for error in the results predicted by the DNN, and the variance of the parameters used as the input to the DNN. In some embodiments, a DNN may even use its own predictions for further training, thus improving the predictions by the DNN over time.

A DNN produces its predictions through a set of mathematical manipulations. Each mathematical manipulation as such is considered a layer, or ply, and complex DNNs have many layers, hence the name "deep" neural networks. DNN architectures generate compositional models where the object is expressed as a layered composition of primitives. The layers in a DNN not being an input layer or an output layer are denoted 'hidden' layers. Beneficially, the layers in a DNN may be fully interconnected, i.e., all nodes in a layer are connected to all nodes in the following layer. Thanks to the increased connectivity between the hidden layers, DNNs can model very complex nonlinear relationships. The extra layers enable composition of features from lower layers, potentially modeling complex data with fewer units than a similarly performing shallow network. It is, for instance, possible to prove that sparse multivariate polynomials are exponentially easier to approximate with DNNs than with shallow networks, i.e., without any hidden layers.

Deep architectures include many variants of a few basic approaches. Each architecture has found success in specific domains. DNNs are typically feed-forward networks in which data flows from the input layer to the output layer without looping back. At first, the DNN creates a map of virtual neurons and assigns random numerical values, or "weights", to the connections between them. The weights and inputs are multiplied and return an output between 0 and 1. If the network does not accurately recognize a particular pattern, a training algorithm is configured to adjust the weights. In that way the algorithm can make certain parameters more influential, until it determines the correct mathematical manipulation to fully process the data leading to a correct prediction.

### SUMMARY

The problem to be solved is to provide a method of fitting a hearing device to a hearing loss automatically while taking both initial fitting settings and additional fine-tuning adjustments into account.

To alleviate the presented problems, it is proposed to utilize data from a plurality of existing hearing device fittings, e.g., from a data pool collected anonymously from a population of hearing device users having had their hearing devices fitted by HCPs, and subsequently build and train a machine learning (ML) model to be trained with the data from said data pool, the data comprising audiograms representing individual hearing losses and corresponding gain prescriptions. A preferred, effective ML model for this specific purpose is a deep neural network (DNN). The inventors have realized that providing some leeway in applying a specific set of gain values to the different frequency bands of a hearing device is beneficial in many situations, e.g., to reduce the amount of unpleasant and unfamiliar sounds from the hearing device for inexperienced users, improve the quality of certain types of speech, or simply adhering to a matter of individual preference of more experienced hearing device users.

In order for the ML model to be able to provide a range of gain values instead of fixed gain settings for different frequency bands it is proposed to construct the ML model as a so-called 'ensemble' or a plurality of DNNs, with each DNN in the ensemble being a little bit different from the other DNNs in the ensemble, thus resulting in slight variations in the gain value predictions from each DNN. After the ML model is trained with data from the data pool, the ML model is then presented with a set of data relating to a prospective hearing device user, the data including an audiogram representing the prospective user's hearing loss and data regarding various details of a proposed hearing device model. The ML model will then be capable of predicting a corresponding set of gains ready to be applied to a particular hearing device, thus enabling fitting the hearing device with the predicted gains.

In a first aspect, this proposal is realized by a method for fitting a hearing device according to claim 1.

Beneficially, the proposed method uses a dataset of real user profiles including real hearing device fittings for training a machine learning algorithm to predict a dataset of gain value ranges from a given user profile. This ensures a coherent and reliable dataset of gain value ranges suitable for fitting a hearing device.

The skilled person will appreciate that the method involves an ensemble of differently configured deep neural networks to predict the desired gain values. In this way, the method makes it possible to predict a range of gain values for a given frequency band in the hearing device instead of a fixed gain value. This ensures flexibility when using the predicted gain range to fit the hearing device by allowing the gain value for a specific frequency band to be slightly higher or lower, only limited by the upper and lower values of the predicted gain range.

In a second aspect, this proposal is realized by a system for fitting a hearing device according to claim 7.

The system may be built into an ordinary hearing device fitting system. This beneficially makes the method readily available to e.g., a healthcare professional (HCP). Once the training procedure has been completed, the HCP may enter a user profile including an audiogram, some personal data, and a proposed hearing device, into the system and get a predicted dataset of gain value ranges from the system as a result. The predicted dataset of gain value ranges may be used as a guideline for fitting a hearing device, or it may be used to check a fitting obtained by other means.

In some embodiments, the system may be built into the hearing device itself. This has the obvious benefit that a user obtaining e.g., an OTC hearing device may enter his or her user profile into the system, and the system may then predict a suitable fitting of the hearing device as a result. In some embodiments, the system may even provide the predicted fitting straight to the hearing device from the user profile data entered directly by the user without other people being involved in fitting the hearing device. In some embodiments, the system may be used to check the current fitting against e.g., a newly obtained audiogram, and perform adjustments if necessary.

### BRIEF DESCRIPTION OF THE DRAWINGS

The invention is now described in greater detail with respect to the drawings, where:
Fig. 1 illustrates a hearing device fitting procedure according to the prior art,
Fig. 2 illustrates a training procedure according to the method,
Fig. 3 illustrates a neural network structure suited for implementing the method,
Fig. 4a illustrates a table of input parameters for a ML model of a fitting system,
Fig. 4b is the table of fig. 4a filled with exemplary user profile data,
Fig. 5 illustrates a system for carrying out the method, and
Fig. 6 is a diagram of an exemplary set of predicted gain ranges for a hearing device.

### DETAILED DESCRIPTION

A hearing device is configured to be worn by a user. The hearing device may be arranged at the user's ear, on the user's ear, over the user's ear, in the user's ear, in the user's ear canal, behind the user's ear and/or in the user's concha, i.e., the hearing device is configured to be worn in, on, over and/or at the user's ear. The user may wear two hearing devices, one hearing device at each ear. The two hearing devices may be connected, such as wirelessly connected and/or connected by wires, such as a binaural hearing aid system.

The hearing device may be a hearable such as a headset, headphone, earphone, earbud, hearing aid, a personal sound amplification product (PSAP), an over-the-counter (OTC) hearing device, a hearing protection device, a one-size-fits-all hearing device, a custom hearing device or another head-wearable hearing device. Hearing devices can include both prescription devices and non-prescription devices. The hearing device may be embodied in various housing styles or form factors. Some of these form factors are Behind-the-Ear (BTE) hearing device, Receiver-in-Canal (RIC) hearing device, Receiver-in-Ear (RIE) hearing device or Microphone-and-Receiver-in-Ear (MaRIE) hearing device. These devices may comprise a BTE unit configured to be worn behind the ear of the user and an in the ear (ITE) unit configured to be inserted partly or fully into the user's ear canal.

Generally, a BTE unit may comprise at least one input transducer, a power source, and a processing unit. The term BTE hearing device refers to a hearing device where the receiver, i.e. the output transducer, is comprised in the BTE unit and sound is guided to the ITE unit via a sound tube connecting the BTE and ITE units, whereas the terms RIE, RIC and MaRIE hearing devices refer to hearing devices where the receiver may be comprised in the ITE unit, which is coupled to the BTE unit via a connector cable or wire configured for transferring electric signals between the BTE and ITE units. Some of these form factors are In-the-Ear (ITE) hearing device, Completely-in-Canal (CIC) hearing device or Invisible-in-Canal (IIC) hearing device. These hearing devices may comprise an ITE unit, wherein the ITE unit may comprise at least one input transducer, a power source, a processing unit, and an output transducer. These form factors may be custom devices, meaning that the ITE unit may comprise a housing having a shell made from a hard material, such as a hard polymer or metal, or a soft material such as a rubber-like polymer, molded to have an outer shape conforming to the shape of the specific user's ear canal. Some of these form factors are earbuds, on-the-ear headphones, or over-the-ear headphones. The person skilled in the art is well aware of different kinds of hearing devices and of different options for arranging the hearing device in, on, over and/or at the ear of the hearing device wearer. The hearing device (or pair of hearing devices) may be custom fitted, standard fitted, open fitted and/or occlusive fitted.

In some embodiments, the hearing device may comprise one or more input transducers. The one or more input transducers may comprise one or more microphones. The one or more input transducers may comprise one or more vibration sensors configured for detecting bone vibration. The one or more input transducer(s) may be configured for converting an acoustic signal into a first electric input signal. The first electric input signal may be an analogue signal. The first electric input signal may be a digital signal. The one or more input transducer(s) may be coupled to one or more analogue-to-digital converter(s) configured for converting the analogue first input signal into a digital first input signal.

In some embodiments, the hearing device may comprise one or more antenna(s) configured for wireless communication. The one or more antenna(s) may comprise an electric antenna. The electric antenna may be configured for wireless communication at a first frequency. The first frequency may be above 800 MHz, preferably a wavelength between 900 MHz and 6 GHz. The first frequency may be 902 MHz to 928 MHz. The first frequency may be 2.4 to 2.5 GHz. The first frequency may be 5.725 GHz to 5.875 GHz. The one or more antenna(s) may comprise a magnetic antenna. The magnetic antenna may comprise a magnetic core. The magnetic antenna may comprise a coil. The coil may be coiled around the magnetic core. The magnetic antenna may be configured for wireless communication at a second frequency. The second frequency may be below 100 MHz. The second frequency may be between 9 MHz and 15 MHz.

In some embodiments, the hearing device may comprise one or more wireless communication units. The one or more wireless communication units may comprise one or more wireless receivers, one or more wireless transmitters, one or more transmitter-receiver pairs and/or one or more transceivers. At least one of the one or more wireless communication unit may be coupled to the one or more antenna. The wireless communication unit may be configured for converting a wireless signal received by at least one of the one or more antennas into a second electric input signal. The hearing device may be configured for wired/wireless audio communication, e.g., enabling the user to listen to media, such as music or radio and/or enabling the user to perform phone calls.

In some embodiments, the wireless signal may originate from one or more external sources and/or external devices, such as spouse microphone devices, wireless audio transmitters, smart computers and/or distributed microphone arrays associated with a wireless transmitter. The wireless input signals may originate from another hearing device, e.g., as part of a binaural hearing system and/or from one or more accessory devices, such as a smartphone and/or a smart watch. In some embodiments, the wireless signal may include settings for programming or fitting the hearing device.

In some embodiments, the hearing device includes a processing unit. The processing unit may be configured for processing the first and/or second electric input signals. The processing may comprise compensating for a hearing loss of the user, i.e., apply frequency dependent gain to input signals in accordance with the user's frequency dependent hearing impairment. The processing may comprise performing feedback cancelation, beamforming, tinnitus reduction/masking, noise reduction, noise cancellation, speech recognition, bass adjustment, treble adjustment and/or processing of user input. The processing unit may be a processor, an integrated circuit, an application, a functional module, etc. The processing unit may be implemented in a signalprocessing chip or a printed circuit board (PCB). The processing unit may be configured to provide a first electric output signal based on the processing of the first and/or second electric input signals. The processing unit may be configured to provide a second electric output signal. The second electric output signal may be based on the processing of the first and/or second electric input signals.

In some embodiments, the hearing device may comprise an output transducer. The output transducer may be coupled to the processing unit. The output transducer may be a receiver. It is noted that in this context, a receiver may be a loudspeaker, whereas a wireless receiver may be a device configured for processing a wireless signal. The receiver may be configured for converting the first electric output signal into an acoustic output signal. The output transducer may be coupled to the processing unit via the magnetic antenna. The output transducer may be comprised in an ITE unit or in an earpiece, e.g., Receiver-in-Ear (RIE) unit or Microphone-and-Receiver-in-Ear (MaRIE) unit, of the hearing device. One or more of the input transducers may be comprised in an ITE unit or in an earpiece.

In some embodiments, the wireless communication unit may be configured for converting the second electric output signal into a wireless output signal. The wireless output signal may comprise synchronization data. The wireless communication unit may be configured for transmitting the wireless output signal via at least one of the one or more antennas.

In some embodiments, the hearing device may comprise a digital-to-analogue converter configured to convert the first electric output signal, the second electric output signal and/or the wireless output signal into an analogue signal.

In some embodiments, the hearing device may comprise a vent. A vent is a physical passageway such as a canal or tube primarily placed to offer pressure equalization across a housing placed in the ear such as an ITE hearing device, an ITE unit of a BTE hearing device, a CIC hearing device, a RIE hearing device, a RIC hearing device, a MaRIE hearing device or a dome tip/earmold. The vent may be a pressure vent with a small cross section area, which is preferably acoustically sealed. The vent may be an acoustic vent configured for occlusion cancellation. The vent may be an active vent enabling opening or closing of the vent during use of the hearing device. The active vent may comprise a valve.

In some embodiments, the hearing device may comprise a power source. The power source may comprise a battery providing a first voltage. The battery may be a rechargeable battery. The battery may be a replaceable battery. The power source may comprise a power management unit. The power management unit may be configured to convert the first voltage into a second voltage. The power source may comprise a charging coil. The charging coil may be provided by the magnetic antenna.

In some embodiments, the hearing device may comprise a memory, including volatile and non-volatile forms of memory. The memory may be used to store processing parameters, e.g., a set of amplification gains for a range of frequency bands for the purpose of alleviating a hearing loss. The set of amplification gains may be a set of prescription amplification gains determined at a fitting session. The memory may also be used to store a set of hearing programs selectable by the user, e.g., one program for processing speech in noise, another program for processing wireless signals received by the antenna in the hearing device, several programs dedicated to processing sound received in different, specific sound environments, and a program for handling telephone conversations. In some embodiments, the memory may be used to store hearing device usage statistics.

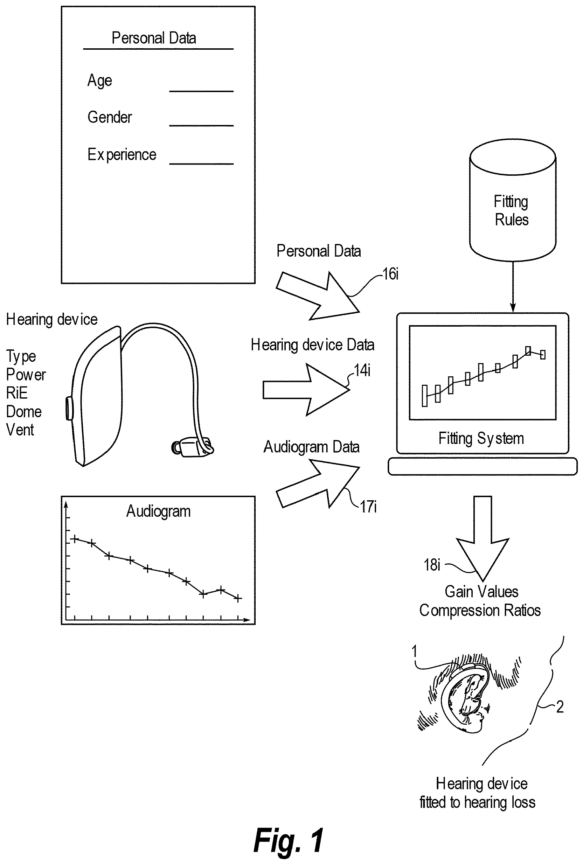

The adaptation of a hearing device to the hearing loss of a specific user involves an HCP adjusting the hearing instrument electronics in such a way that sounds picked up by the hearing device microphones are sufficiently amplified to be perceived effortlessly by the user. This procedure is known as 'fitting the hearing device' and is illustrated in fig. 1. When a prospective hearing device user 2 presents an HCP with a hearing problem, the HCP obtains an audiogram of the prospective user's unaided hearing capabilities and possible hearing loss in preparation for the fitting session. The audiogram may e.g., be a pure-tone audiogram taken over a range of different, discrete frequencies within the audible frequency spectrum. The audiogram data 17i reflects the prospective hearing device user's hearing threshold for a predetermined set of discrete frequencies. The audiogram may be provided at a session separate from the actual fitting session.

Prior to initiating the fitting session, the HCP collects various personal data 16i relating to the prospective user 2, including information relating to hearing loss, age, gender, and former hearing device experience, and may also include e.g., a medical history, results of an ear examination, and subjective hearing problems suffered by the prospective user 2.

The HCP then selects a `blank' hearing device 1 of a suitable type based on the data collected for the prospective user 2, i.e., a hearing device which have not yet been fitted to a specific hearing loss. Data 14i regarding the selected hearing device 1 includes a hearing device form factor (e.g., behind-the-ear, in-the-ear, in-the-canal, receiver-in-ear), a receiver power range (e.g., normal power, high-power), an ear canal dome or earmold type and size according to the size and shape of the ear canal of the user 2 (e.g., small, medium, large, custom-molded), a sound tube length (if not a receiver-in-the-ear, in-the-ear or in-the-canal hearing device type), and finally, the applicability and eventual size of an earmold vent (e.g., none, small, medium, large) The hearing device data 14i defines the adjustment capabilities and limits of the hearing device 1.

Utilizing the personal data 16i, the audiogram data 17i, and the hearing device data 14i concerning the selected hearing device 1, the HCP uses his or her experience and selects one or more predetermined fitting rules in order to apply individual amplification gains to the hearing device 1 over a range of predetermined, individual frequency bands using a hearing device fitting system connected to the hearing device 1 during the fitting session to compensate for a hearing loss. The fitting system may be a dedicated piece of hardware capable of connecting wired or wirelessly to the hearing device during fitting, or it may be a hearing device fitting program executed on e.g., a personal computer equipped with a suitable data communication interface (not shown in fig. 1) to allow communication with the hearing device circuitry for the purpose of programming the hearing device 1 with suitable, individual amplification gains for alleviating a hearing loss of the user 2.

The purpose of applying a specific amplification gain to a particular frequency band in the hearing device 1 is to increase the level of weaker sounds in that particular frequency band to a level above the user's hearing threshold in that frequency band. The amplification needs to be sufficiently high for the user 2 to be able to perceive sound in that frequency band while avoiding a situation where the level of the amplified sound exceeds e.g., an upper comfort level of the user 1. This task is performed by a signal processor (not shown in fig. 1) or amplifier aboard the hearing device 1, e.g., by amplifying softer sounds more and amplifying louder sounds less in that particular frequency band, typically by using some form of dynamic level compression of that particular frequency band in the hearing device 1. During the fitting session, this process is repeated for all the frequency bands available to the hearing device processor, resulting in a tailored set of amplification gains for the different frequency bands to be applied to the hearing device 1.

The selected fitting rule may take the challenge of reproducing sounds at varying dynamic levels into account by calculating suitable gain values for e.g., three different sound input levels such as 50 dB, 65 dB and 80 dB for fitting each frequency band of the hearing device 1, thus adjusting the hearing device 1 to amplify e.g., a sound input level around 50 dB more than a sound input level around e.g., 80 dB in a particular frequency band, thereby providing dynamic compression of the signals amplified by the hearing device 1. Different fitting rules may devise different settings to be applied by the HCP when fitting the hearing device 1, but usually provides some leeway for the HCP to deviate slightly from the settings devised by a particular fitting rule. These deviations may, e.g., be based on the experience of the HCP, attention to special listening situations often experienced by the user 2, or particular preferences of the hearing device user 2.

Once the HCP has found suitable amplification gain settings for the particular hearing device 1, the gain settings are then transferred to memory of the particular hearing device 1 using the data communication interface. The amplification gain settings to be applied to a hearing device is denoted prescription gain settings. For brevity, the term 'gain settings' is used interchangeably.

To conclude the fitting session, the HCP usually performs a series of listening tests involving the hearing device user 2 in order to ascertain whether the user is satisfied with the hearing device settings applied, and, in the negative, perform fine-tuning adjustments to the prescription gain settings applied to the hearing device 1. Depending on the type of hearing loss to be alleviated by the hearing device 1, this fine-tuning procedure can sometimes be very time-consuming and cumbersome, often involving repeated visits to the HCP over a period of several months after applying the initial first fit of the hearing device 1 to the user 2. An improved method for providing a good, first fit of a hearing device to a person's hearing loss is therefore desired.

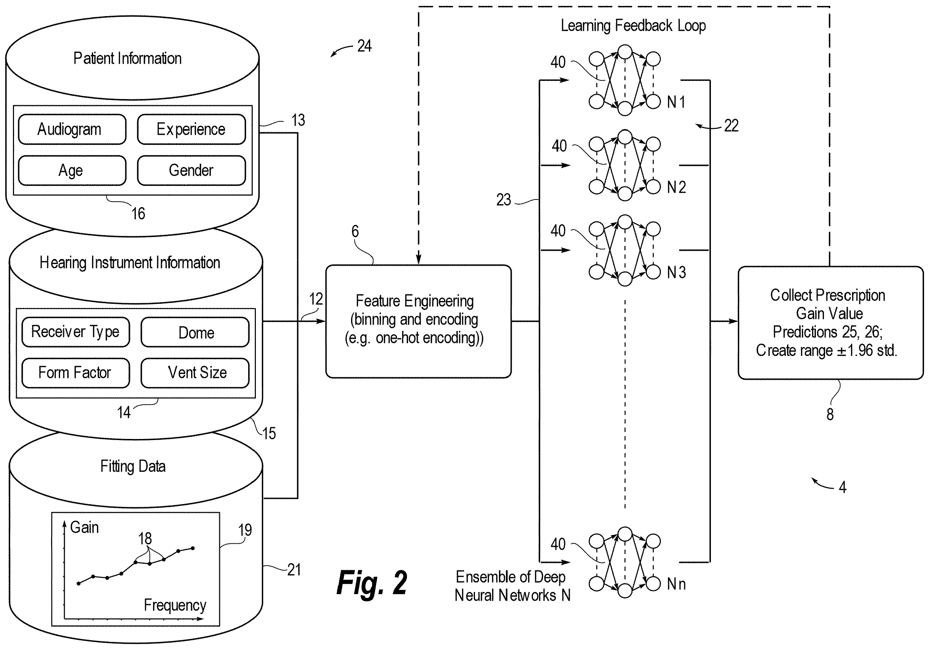

Fig. 2 illustrates the working principle of a system 4 for fitting a hearing device according to the method. The system 4 comprises an ensemble 23 of fitting models 22. Each fitting model 22 is embodied as a Deep Neural Network (DNN) 40. Each fitting model 22 is configured for taking a set of input parameters related to a hearing loss and predict a set of output parameters related to a particular setting in a hearing device. In fig. 2 is shown four DNNs 40 numbered N1, N2, N3,..., Nn. The DNNs 40 are numbered because each DNN 40 is slightly different from the other DNNs 40 in the ensemble 23. The purpose of this difference will be explained later in this disclosure.

In an exemplary embodiment of the system 4, the number n of DNNs 40 in the ensemble 23 is fifteen. In another exemplary embodiment, the number n of DNNs 40 in the ensemble 23 is ten. In some embodiments, the physical realization of the ensemble 23 of DNNs 40 is embodied as one single, physical DNN 40, where the node parameters and weights are stored in memory and exchanged for realizing each DNN 40 in turn, with e.g., the numbers N1, N2 and N3, respectively. In this way, computing resources may be conserved, since only one physical network structure needs to be active at any given moment during use of the system 4.

Prior to use, the system 4 is trained in a training procedure using a training dataset 24 comprising a plurality of user profile datasets 12 including a plurality 13 of user datasets 16, a plurality 15 of corresponding hearing device datasets 14, and a plurality 21 of corresponding gain value datasets 19. Each gain value dataset 19 in the training dataset 24 comprises a plurality of gain values 18 for a set of frequency bands to be amplified by the corresponding hearing device of the hearing device dataset 14. In some embodiments, the number of gain values 18 in each gain value dataset 19 is nine gain values. Each hearing device dataset 14 in the training dataset 24 is thus linked to the corresponding user dataset 16 via the particular hearing device fitting in the form of the corresponding gain value dataset 19 provided to that particular user. The training procedure is preferably implemented as a supervised learning procedure.

The training dataset 24 is collected anonymously from data of actual hearing device users having been fitted with actual hearing devices provided with actual gain values for the purpose of alleviating hearing loss. The fittings have preferably been carried out according to the procedure outlined in fig. 1. The plurality of user profile datasets 12 of the training dataset 24 used to train the DNNs 40 embodying the fitting models 22 of the system 4 preferably represents the latest hearing device fitting available in each case, thereby taking any subsequent fine-tuning of the hearing device settings into account when preparing the training dataset 24. Prior to applying the training dataset 24 to the ensemble 23 of the fitting models 22, the data in the training dataset 24 is sorted, binned, and encoded in an input 6 to the system 4 to ensure uniformity of the input features used during the training procedure, and each node weight (not shown in fig. 2) of each node in each DNN 40 is assigned a random, initial value, thus creating a different starting point for each DNN 40 prior to training.

During the training procedure, each of the user profile datasets 12 in the training dataset 24 are presented to each of the fitting models 22 in turn, and each DNN 40 then produces a resulting dataset 25 of intermediate gain values 26. The number of intermediate gain values 26 predicted is equal to the number of actual gain values 18. The intermediate gain values 26 of the dataset 25 are then presented to an output 8 of the system 4 and compared against the actual gain values 18 of the corresponding gain value dataset 19 using a learning feedback loop, as indicated in fig. 2. The node weights (not shown in fig. 2) of the nodes in each DNN 40 are then adjusted in order to minimize the prediction error between each of the intermediate gain values 26 and each of the corresponding actual gain values 18 predicted by each DNN 40. This error minimization is carried out using e.g., a steepest gradient descent-method or similar error-minimizing function. The training procedure repeats the learning feedback loop for that intermediate gain value 26 until a predetermined satisfaction condition is met, e.g., when the prediction error is below a predetermined, desired prediction confidence level. When the satisfaction condition is fulfilled, the training procedure continues by comparing the next intermediate gain value 26 to the corresponding next actual gain value 18, and the node weights of the nodes in the DNN 40 are adjusted again. The training procedure is then repeated for all user profile entries 12 in the complete training dataset 24 and all for the fitting models 22 in the ensemble 23, adjusting the node weights of each DNN 40 accordingly after each pass.

It should be noted that the satisfaction condition alternatively could be that the learning feedback loop has been repeated a specific number of times, e.g., 32 times, or that the absolute error between intermediate gain value 26 and the corresponding actual gain value 18 is below a specific value, or that the prediction error starts increasing again after having reached a minimum value. The satisfaction condition may be selected with respect to e.g., the desired accuracy of the predictions, the time available to traverse the training dataset 24 during the training procedure, and the chosen step size with which the node weights in the DNN 40 is adjusted.

During the training procedure, each DNN 40 thus improves its predicted values of the dataset 25 of intermediate gain values 26 for a particular user profile entry 12 with respect to the corresponding dataset 19 of the actual gain values 18, and the prediction error thus decreases until the error between each of the actual gain values 18 of the training dataset 24 and each of the corresponding intermediate gain values 26 produced by each DNN 40meets a satisfaction condition. The training procedure is then completed, and the values of the node weights assigned to each node in each DNN 40 of the ensemble 23 of fitting models 22 are fixated.

Preferably, the system 4 uses a relatively large training dataset 24 to train the ensemble 23 of fitting models 22 in order to ensure a sufficiently high confidence in subsequent predictions to be performed by the system 4. In an embodiment, a training dataset 24 comprising around 350.000 individual data values, representing about 9.000 individual hearing device fittings, is used to train the ensemble 23 of fitting models 22. The size of the training dataset 24 is exemplary, and a larger training dataset 24 of e.g., 500.000 or perhaps 1.000.000 individual data values could, theoretically, permit the system 4 to provide even more accurate predictions.

Beneficially, every fitting model 22 in the ensemble 23 is slightly different from the other fitting models 22 in the ensemble 23, partly due to the random initial values assigned to the node weights prior to training, and partly due to other factors such as data shuffling and various design choices, such as optimizers and regularization techniques known to the skilled person. Inherently, the DNNs N1, N2, N3, etc., representing the fitting models 22, therefore gives slightly different, albeit similar gain value predictions. The benefit achieved by the system 4 exhibiting these differences is explained in the following.

The benefit of having an ensemble 23 of different fitting models 22 is that this gives the system 4 the capability of predicting a range of gain values for each frequency band in a hearing device instead of predicting just one, fixed gain value for each frequency band. By having the system 4 provide a range of fitting gains for each frequency band, an HCP may use the system 4 to predict a first fitting for a hearing device while maintaining a degree of freedom to select a value from within the predicted gain range for each frequency band for fitting the hearing device, e.g., according to a specific preference of a user, knowledge of a range of listening environments in which the hearing device is going to be used, or permit use of the system 4 to provide a confirmation interval of fitting gains to check the validity of, e.g., a hearing device fit crafted by the HCP using a given fitting rule.

In an alternative embodiment, the architecture of each DNN 40 in the ensemble 23 of fitting models 22 may be different, so that a first DNN N1 in fig. 2 may have two hidden layers, where the first hidden layer has seventy-eight nodes and the second hidden layer has twelve nodes, whereas a second DNN N2 in fig. 2 may have two hidden layers, where the first hidden layer has seventy-six nodes, and the second hidden layer has seventeen nodes. A third DNN N3 in fig. 2 may have three hidden layers, where the first hidden layer has eighty-two nodes, the second hidden layer has one-hundred-and-seven nodes, and the third hidden layer has twenty-one nodes. This embodiment may create a different spreading in the resulting predictions made by each fitting model 22, should such a spreading pattern be desired, however, it may be more complicated to implement.

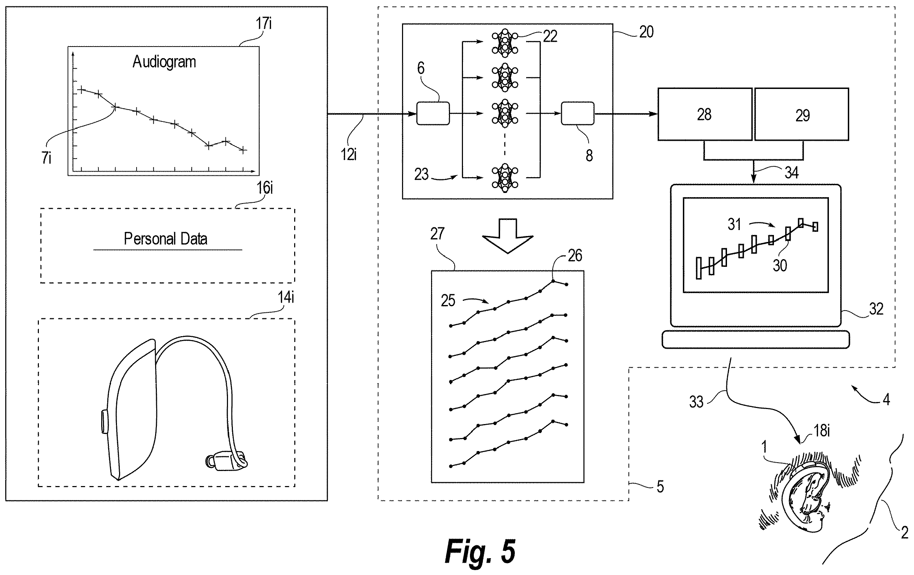

In an exemplary embodiment, the number n of DNNs 40 in the ensemble 23 in the system 4 is fifteen. In other words, fifteen different fitting models 22 are employed to predict fifteen datasets 25 of intermediate gain values 26 in this embodiment. The benefit of having, e.g., fifteen fitting models 22 predict the dataset 25 of intermediate gain values 26 is that each of the fifteen datasets 25 of intermediate gain values 26 will be slightly different from one another while maintaining audiological validity. The advantage of this approach is that when these differences are processed statistically the result is a dataset of resulting gain value ranges, each gain value range expressing both an upper gain value and a lower gain value for each frequency band in the hearing device to be fitted. The implications of this approach will be discussed in greater detail in the following with reference to fig. 5.

When the training procedure of the ensemble 23 of fitting models 22 is completed, the system 4 is ready to output a predicted dataset of resulting gain value ranges via the output 8 of the system 4 when the system 4 is presented with an individual user profile dataset 12i (not shown in fig. 2) on the input 6.

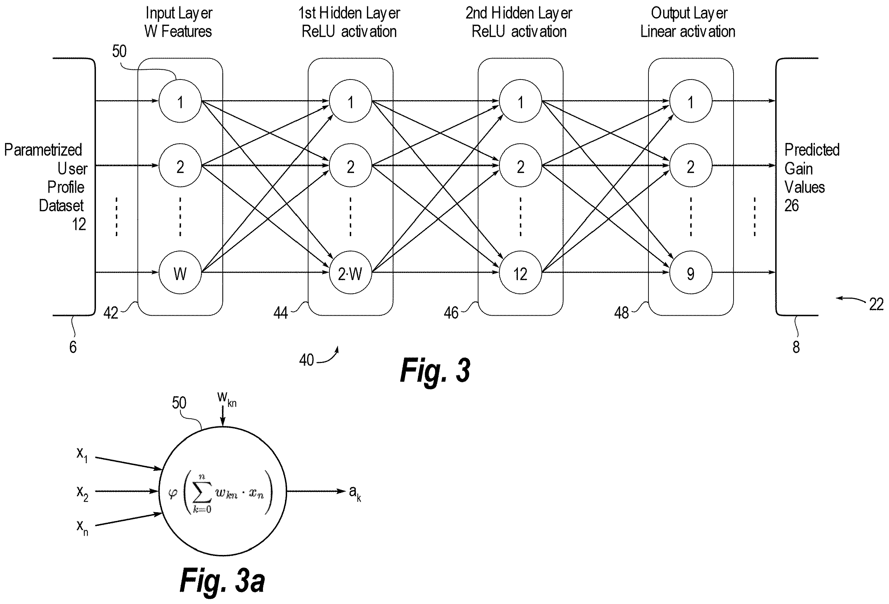

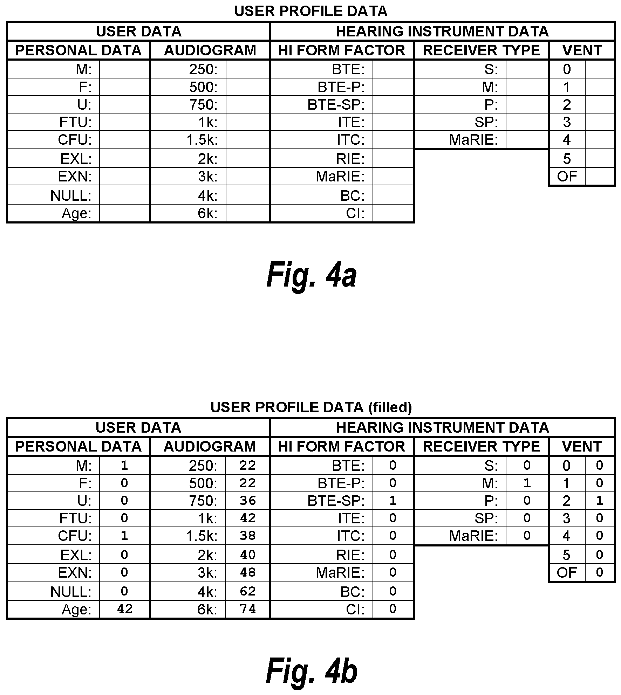

Fig. 3 illustrates an exemplary DNN 40 configured for implementing a fitting model 22 as part of the method. The exemplary DNN 40 in fig. 3 is embodied as a 4-ply, fully connected, neural network, and comprises an input layer 42, a first hidden layer 44, a second hidden layer 46, and an output layer 48. Each layer 42, 44, 46, 48 comprises a predetermined number of nodes 50, where each node 50 comprises at least one input, at least one weight, and at least one output, as shown in fig. 3a. All nodes in the input layer 42 have only one input, and all nodes in the DNN 40 have only one output. This output is, however, presented to the input of every node in the subsequent layer. Each input of each node of the input layer 42 is configured to take a specific parameter of a parametrized user profile dataset 12, i.e., audiogram level values at different frequencies, the user's age, gender, and hearing device experience level, various other relevant user data, and data relating to the hearing device to be fitted, i.e., hearing device form factor, receiver type, vent size etc., as its input. The parametrization is explained in greater detail in the following with reference to the table in fig. 4a.

All the node outputs of the input layer 42 are thus connected to every node input of a second number of nodes of the first hidden layer 44. The number of nodes W in the input layer 42 corresponds to the number of input parameters of the parametrized user profile dataset 12, i.e., exemplary between thirty-five and forty nodes, and the number of nodes in the first hidden layer 44 is preferably double the number of nodes in the input layer 42, i.e., 2·W, or exemplary, seventy to eighty nodes. All the node outputs of the nodes in the first hidden layer 44 are connected to every node input of a third number of nodes in the second hidden layer 46. The number of nodes in the second hidden layer 46 is preferably between twelve to fourteen nodes. In this exemplary DNN 40, the structure follows a scale-up, funnel-down architecture, but other structures are equally valid, e.g., a pure funnel-down architecture of thirty-five nodes in the input layer 42, twenty-four nodes in the first, hidden layer 44, and nine nodes in the second, hidden layer 46, or a flat architecture with thirty-five nodes in the input layer 42, thirty-five nodes in the first hidden layer 44, and thirty-five nodes in the second hidden layer 46. In every case, the layers 42, 44, 46 and 48 in the DNN 40 are fully connected. The nodes in the first hidden layer 44 and the second hidden layer 46 preferably uses a Rectified Linear Unit (ReLU) function for activation, primarily in order to ensure efficacy during the training procedure. Alternatively, another suitable function, such as a sigmoid function, may be used for activation. All the outputs of the nodes in the second hidden layer 46 are connected to the inputs of the nodes in the output layer 48. The number of nodes in the output layer 48 corresponds to the number of frequency bands for which gain value ranges are to be estimated, typically nine nodes in some preferred embodiments.

As stated in the foregoing, each node 50 in each layer 42, 44, 46, 48 of the DNN 40 in fig. 3 has a plurality of inputs, a plurality of corresponding weights, and an output. Each parameter in the parametrized user profile dataset is applied to the input of one node in the input layer 42. Each output of each of the nodes in the input layer 42 is connected to an input of each of the nodes in the first hidden layer 44, and the weights in each node of the first hidden layer 44 are multiplied with the corresponding node input values, each of the weights being a number between 0 and 1. Each output of each node in the first hidden layer 44 is connected to an input of each of the nodes in the second hidden layer 46 and carries the rectified, linear output values of each of the nodes in the first hidden layer 44. In the second hidden layer 46, the weights in each node are multiplied with the corresponding node input values. Similarly, each output of each node in the second hidden layer 46 is connected to an input of each of the nodes in the output layer 48 and carries the rectified, linear output values of each of the nodes in the second hidden layer 46. In the output layer 48, each node weight of each node in the output layer 48 is multiplied with the input value in each node. Each of the nodes in the output layer 48 is then configured, by the training procedure, to provide an intermediate gain value 26 estimated or predicted by the DNN 40 for a particular frequency band of a hearing device and presents the intermediate gain values at its output 8. The output layer 48 uses a linear identity function for activation.

An exemplary, single node 50 of a layer in a DNN 40 is illustrated in fig. 3a. The node 50 has a plurality of inputs x₁, x₂...xₙ, a set of weights wₖₙ (only one weight shown in fig. 3a) and an output aₖ. The node output aₖ of the k^{th} node of the hidden layers 44 and 46 is described by the following function: ${a}_{k} = ϕ \left({\sum }_{k = 0}^{n} {w}_{kn} ⋅ {x}_{n}\right) ; ϕ \left(x\right) = max \left(0, x\right)$

Where k is the input index from the previous node and ϕ is the activation function, embodied as a rectified linear unit function in the hidden layers 44 and 46.

The node output aₖ of the k^{th} node of the output layer 48 is described by the following function: ${a}_{k} = ϕ \left({\sum }_{k = 0}^{n} {w}_{kn} ⋅ {x}_{n}\right) ; ϕ \left(x\right) = x$

The only difference between the nodes of the hidden layers 44 and 46, and the nodes of the input layer 42 and the output layer 48 is that the activation function ϕ in the nodes in these layers is the identity function.

During training of the DNN 40, a parametrized user profile dataset 12 for each entry in the training dataset 24 is applied to the input layer 42 of the DNN 40 via the input 6 as illustrated in fig. 3 in the manner discussed in the foregoing, and a corresponding dataset of predicted, intermediate gain values 26 is presented at the output 8 of the DNN 40. Each of the intermediate gain values 26 for each user profile dataset 12 is then compared to each of the actual gain values 18 associated with the corresponding user profile dataset 12, and in a learning feedback loop, the values of all the weights of all the nodes of all the layers of the DNN 40 are adjusted accordingly in order to minimize the error between the intermediate gain values 26 and the actual gain values 18. The learning feedback loop may e.g., use a backpropagation algorithm in the training procedure. In an embodiment, the adjustment of the weights in the DNN 40 may follow a steepest-gradient-descent convergence scheme. In alternative embodiments, the adjustment of the weights in the DNN 40 may follow a least-mean-square convergence scheme or a similar error minimizing function known to the skilled person. When the error between each predicted, intermediate gain value 26 of a particular user profile dataset 12 and the corresponding, actual gain value 18 is smaller than a predetermined minimal difference, the training procedure continues with the next user profile dataset 12 in the training dataset 24 until all the user profile datasets 12 of the training dataset 24 has been applied to the DNN 40 and the weights of all the nodes have been adjusted accordingly.

Preferably, the training dataset 24 is applied to the system 4 during the training procedure in small batches of user profile datasets 12, by bundling e.g., fifty to one hundred user profile datasets 12 in each batch, thus limiting the amount of value adjustment applied to each weight in the DNN 40 while optimizing for mean square error per batch. This limitation in weight value adjustment provides for a slower, albeit more robust prediction convergence. The prediction and weight value adjustment feedback process is repeated until a satisfaction criterion is met, e.g., that the error between the intermediate gain value 26 and the actual gain value 18 for a particular frequency band in a particular user profile dataset 12 is deemed to be sufficiently small. This procedure is then repeated for all the user profile dataset entries 12 in the training dataset 24 and for every fitting model 22 in the ensemble 23. The plurality of DNNs 40, individually denoted N1, N2, N3...Nn in fig. 2, represents the ensemble 23 of different fitting models 22, and every DNN 40 is thus trained individually with the complete training dataset 24. Once all the DNNs 40 have been trained in this way, each weight of each node in each layer 42, 44, 46, 48 of every DNN N1, N2, N3...Nn of the ensemble 23 of fitting models 22 is locked or fixed at its current value, and each fitting model 22 is ready to predict a set of individual, intermediate gain values suitable for fitting a particular hearing device.

Fig. 4a illustrates, in table form, an exemplary set of parametrized user profile data for use as input data for the system 4, either in the form of a user profile dataset entry 12 of the training dataset 24 or in the form of an individual user profile 12i entered into the trained system 4 for predicting gain value ranges. The table in fig. 4a has five columns covering the user data categories 'Personal Data', and 'Audiogram', and the hearing instrument data categories 'Hearing Instrument Form Factor', 'Receiver Type', and 'Vent Size'. Some entries in the table, like gender or form factor, are implemented as one-hot encoded, binary data types, while other entries, like age or hearing threshold level, are implemented as numeric types. It should be stressed with respect to the method that the exemplary tables in fig. 4a and 4b are included for illustrative purposes only in order to illustrate how data may be effectively organized before being presented to the system 4. The data categories illustrated are also non-exhaustive.

The first user data column in the table in fig. 4a, covering the data category 'Personal Data', includes the binary types 'Gender' (taking the binary values: Male; Female; Unknown) and 'Experience Level' (taking the binary values: FTU; CFU; EXL; EXN; NULL), and the numeric type `Age' (taking the numeric values: 0-120 years, 0 years meaning 'unknown age'). The type 'Gender' is self-explanatory, and the type 'Experience Level' has five, mutually exclusive, possible choices relating to the hearing device experience level of the user.

The first possibility, `FTU', indicates e.g., that the user is a "First-Time User" who has not yet been fitted with a hearing device, the possibility `CFU', indicates e.g., that the user is a "Comfort User", who has been using a hearing device for some time, e.g., under a year, the possibility `EXL', indicates that the user has experience using a 'linear' hearing device, i.e. a hearing device without dynamic compression, thus being an "Experienced-Linear" user, the possibility `EXN', indicates that the user has experience using a hearing device with dynamic compression enabled, thus being an "Experienced-Nonlinear" user, and the last possibility `NULL' indicates that no user information about prior hearing device use is known. This is of some importance, e.g., when fitting a prospective hearing device user with a hearing device for the very first time. In that case, it is common for a first fitting to provide a bit less than ideal amplification in order to let the user adapt to the experience of wearing and using a hearing device on a daily basis. A hearing device user may also be initially uncomfortable with a hearing device setting involving dynamic compression if the user hitherto only has had experience using a hearing device without dynamic compression, thus the categories `EXL' and 'EXN'. Since the training dataset 24 comprises a percentage of e.g., first-time users, the system 4 is trained to take the experience level into account when predicting the gain value ranges. The numeric type `Age' (in years) may similarly be utilized to take an age-related hearing loss into account when fitting a hearing device by having a percentage of age-related hearing loss compensation profiles available in the training dataset 24.

The second user data column in the table in fig. 4a covers the data category 'Audiogram' and includes the numeric type 'Hearing Threshold' at different, audible frequencies, (taking the numeric values: -10 dB - 120 dB) indicating a prospective hearing device user's hearing threshold level in decibels at a number of fixed frequencies in the audible frequency spectrum, preferably as the result of a pure-tone hearing ability test at a number of different frequencies, either performed by the HCP at an initial consultation or by using, e.g., an online hearing test service such as an automated Bayesian-Pure-Tone-Audiometry (BPTA) test or the like. The hearing threshold level indicates the softest level the prospective hearing device user is capable of perceiving unaided at each fixed frequency. A common set of fixed frequencies may e.g., include the frequencies 250 Hz, 500 Hz, 750 Hz, 1 kHz, 1,5 kHz, 2 kHz, 3 kHz, 4 kHz, and 6 kHz, but a subset of these frequencies, or a set of different frequencies, may alternatively be used instead. Beneficially, these values may be taken directly from the audiogram of the prospective hearing device user.

The first hearing instrument data column in the table in fig. 4a covers the data category 'Hearing Instrument Form Factor' and includes the mutually exclusive, binary types 'Behind-The-Ear' (BTE), 'Behind-The-Ear-Power' (BTE-P), 'Behind-The-Ear-Super-Power' (BTE-SP), 'In-The-Ear' (ITE), 'In-The-Canal' (ITC), 'Receiver-In-Ear' (RIE), 'Microphone-and-Receiver-In-Ear' (MaRIE), 'Bone-Conductive' (BC), and 'Cochlear-Implant' (CI). A hearing device from the set of available hearing device types is usually selected by the HCP when an audiogram has been obtained, using the audiogram results as a rough guideline for selecting the most suitable and capable hearing device type for the prospective user while taking desires and expectations of the prospective hearing device user into account.

The second hearing instrument data column in the table in fig. 4a covers the data category 'Receiver Type', and includes the mutually exclusive, binary types 'Small' (S), 'Medium' (M), 'Power' (P), 'Super-Power' (SP), and 'Microphone-and-Receiver-In-Ear' (MaRIE). The receiver type is usually chosen after a hearing device type has been selected, since not all receiver types are suitable for every hearing device type. The hearing device form factor types 'Bone-Conductive' (BC), and 'Cochlear-Implant' (CI) are special cases since these hearing device types do not include ordinary receivers. Whenever one of these form factors are chosen, no receiver type is required.

The third hearing instrument data column in the table in fig. 4a covers the data category 'Vent Size' and includes a range of mutually exclusive, binary types representing standardized vent sizes ranging from 0, i.e., no vent, over 1, 2 and 3, representing smaller vent sizes, to 4 and 5, representing larger vent sizes. The range of vent sizes also includes an 'Open Fit', which is typically used when compensating mild to moderate hearing losses. Using an open fit in a hearing device eliminates the so-called occlusion effect, where the user experiences that the ears are 'clogged' when wearing the hearing device. In hearing devices with very high-power receivers and large gains, larger vent types are usually excluded, or no vent is provided, in order to avoid feedback problems.

The parametrized user profile data table may be implemented directly as part of a user-interface in e.g., the software executing the fitting algorithm or the method of the system 4 permitting entry of the parametrized data directly into the system 4, or it may be embodied as a physical table filled out by hand, e.g., either by the HCP or the prospective user for entry into the system 4 at a more convenient time. It should be stressed once again that the data categories and data types shown in the table in fig. 4a are exemplary, and alternative embodiments may, for instance, include different, parametrized data relevant for providing the system 4 with a sufficient dataset for predicting a suitable fitting of a hearing device without deviating significantly from the method. Alternative, parametrized datasets may e.g., include data regarding an ear canal dome or earmold type and size, a sound tube length and/or thickness (for BTE form factor hearing devices), dynamic level compression settings including desired attack/release times, a bone conducting audiogram, or other similar, relevant data.

Fig. 4b illustrates, in table form, the data of an exemplary, parametrized user profile filled into the blank spaces shown in the table in fig. 4a. From the exemplary user profile, the following facts may be deduced with reference to the explanation of the table entries in fig. 4a: The user is a 42-year-old male being a 'Comfort User' of a hearing device; The audiogram data indicates presence of a hearing loss, especially in the higher frequencies; In this particular case, an in-the-ear hearing device with a 'medium' type receiver and a size 2 vent is initially selected.

When an individual user profile dataset structured as shown in the table in fig. 4b is entered into the system 4 shown in fig. 2 after the ensemble 23 of fitting models 22 has been trained by executing the training procedure using the training dataset 24 as described in the foregoing, the system 4 is configured to automatically predict a dataset of gain value ranges suitable for fitting a hearing device to a user matching the user profile represented by the user profile dataset as explained in greater detail with reference to fig. 5.

Fig. 5 is a block schematic illustrating an embodiment of the system 4 for employing the method. A processor 5 of the system 4 executes a machine learning algorithm 20 comprising an ensemble 23 of fitting models 22. Every fitting model 22 of the ensemble 23 is trained with a training dataset 24 during the training procedure discussed in the foregoing with reference to figs. 2 and 3.

Once the training procedure has been completed, the machine learning algorithm 20 of the system 4 is presented with an individual user profile dataset 12i, relating to a user 2, via the input 6, the individual user profile dataset 12i comprising an individual user dataset 16i including personal data, an individual audiogram 17i comprising a plurality of audiogram datapoints 7i, and a particular hearing device dataset 14i. The individual user profile dataset 12i is preferably organized in a manner similar to the structure shown in the tables in fig. 4a and fig. 4b. When presented with the individual user profile dataset 12i, each of the fitting models 22 of the ensemble 23 then predicts a dataset 25 of intermediate gain values 26 for the different frequency bands in the hearing device 1 and subsequently presents the predicted, intermediate gain values 26 as a plurality 27 of intermediate gain value datasets 25 to an output 8, wherein each intermediate gain value dataset 25 comprises a plurality of intermediate gain values 26 after performing the prediction. Due to the slight difference between each of the fitting models 22 in the ensemble 23 described in the foregoing, each predicted, intermediate gain value dataset 25 in the plurality 27 of intermediate gain value datasets 25 will end up having intermediate gain values 26 being slightly different from one another.

The intermediate gain values 26 thus have a certain spreading between the gain values predicted for each frequency band by each fitting model 22 in the ensemble 23. In an exemplary fitting prediction performed by the system 4, one fitting model 22 may predict an intermediate gain value 26 of 28 dB for the 1 kHz band, while another fitting model 22 may predict an intermediate gain value 26 of 32 dB for the 1 kHz band, thus predicting two different, intermediate gain values 26 for the 1 kHz band. Similarly, the different fitting models 22 will predict slightly different intermediate gain values 26 for the other frequency bands, thereby predicting the plurality 27 of slightly different, but still valid, datasets 25 of intermediate gain values 26.

In order to exploit the differences between the intermediate gain values 26 predicted by each of the fitting models 22, all the datasets 25 are subjected to various statistical analysis methods described in the following. For the purpose of statistical analysis, a mean value block 28 of the processor 5 is provided for calculating mean values of the intermediate gain values 26 in the plurality 27 of datasets 25 for each frequency band, and a standard deviation block 29 of the processor 5 is provided for calculating standard deviations of the intermediate gain values 26 in the plurality 27 of datasets 25 for each frequency band. The calculations from the blocks 28 and 29 subsequently produce a set of statistical values 34 from which a dataset 31 of resulting gain value ranges 30 is subsequently calculated, the dataset 31 of resulting gain value ranges 30 covering all the frequency bands for which a hearing device 1 is to be fitted. The dataset 31 of resulting gain value ranges 30 is then output by the system 4 in a sensible manner, e.g., presented on a terminal display 32. Each gain value range 30 for each frequency band comprises a lower gain limit value, an upper gain limit value, and a plurality of gain values lying between the lower gain limit value and the upper gain limit value.

The mean value block 28 is configured to calculate a mean value for each gain value range 30 for each frequency band from each dataset 25 of intermediate gain values 26, the number of datasets 25 in the plurality of datasets 27 corresponding to the number of fitting models 22 in the ensemble 23 of the machine learning algorithm 20, in the exemplary embodiment, fifteen datasets 25 are predicted in the plurality of datasets 27. The mean value for each gain value range 30 may be calculated by the mean value block 28 as e.g., an arithmetic mean, a geometric mean, a power mean, a harmonic mean or by any similar, suitable mean value calculation known to the skilled person. For the sake of simplicity, an arithmetic mean is used in the following discussion.

An arithmetic mean is defined by: $A = \frac{1}{n} {\sum }_{i = 1}^{n} {a}_{i}$ where aᵢ is a gain value and n is the number of datasets 25, i.e., in this exemplary case, fifteen. When the mean value block 28 is presented with the fifteen gain values predicted by each of the fifteen fitting models 22 in the ensemble 23 for a particular frequency band as an input, the mean value block 28 provides the mean gain value for that particular frequency band as a result. Provided with all the intermediate gain values 26 of all the datasets 25, the mean value block 28 will thus give out a complete set of mean gain values for all the frequency bands in the hearing device to be fitted.

The variance between the datasets 25 of intermediate gain values 26 expresses the range of gain values between which the expected 'best fit' lies. The variability of the gain values is calculated as a standard deviation in the standard deviation block 29 of the system 4.

A standard deviation may, e.g., be calculated as: $σ \left(r\right) = \sqrt{\frac{1}{N - 1} {\sum }_{i = 1}^{N} {\left({x}_{i}-r\right)}^{2}}$ where N is the number of datasets 25, i.e., fifteen in this exemplary case, r is the mean gain value, and xᵢ is the current gain value. The standard deviation o(r) may then be used to assess how much a specific gain value for a particular frequency band may deviate from the calculated mean gain value for that frequency band while still being a valid, fitted gain value as predicted by the system 4. The mean values and standard deviations calculated by the mean value block 28 and the standard deviation block 29, respectively, are used by the system 4 as a statistical value 34 for calculating the dataset 31 of resulting gain value ranges 30. The mean value block 28 and the standard deviation block 29 may be algorithms executed by the processor 5.

The dataset 31 of resulting gain value ranges 30 may be utilized in several different ways which will be described in the following with reference to figs. 2 and 5.

In a first use case, the gain values calculated by the mean value block 28 may be applied directly to a hearing device 1 as individual gain values 18i in a fitting procedure 33 for fitting the hearing device 1 to a user 2.

In a second use case, the HCP may use the dataset 31 of resulting gain value ranges 30, e.g., as presented by the terminal 32, as a guideline for fitting a hearing device 1 to a user 2, or as a means of checking the validity of a proposed hearing device fitting obtained by using e.g., a known fitting rule, and subsequently checking this hearing device fitting against a dataset 31 of resulting gain value ranges 30 provided by the system 4 to ascertain if the gain values obtained by the known fitting rule lies within the limits of the resulting gain value ranges 30 predicted by the system 4. This may also be useful if e.g., a new fitting rule is to be adapted.

In a third use case, a prospective hearing device user 2 may obtain an OTC hearing device and perform a fitting of this OTC hearing device himself or herself using the system 4 without involving an HCP for the first fit of the OTC hearing device. In an embodiment, the system 4 may, e.g., be executed as an application on a consumer-grade device such as a smartphone or a personal computer connecting to the OTC hearing device using e.g., a wired connection, such as USB, or a wireless connection, such as Bluetooth. In an alternative embodiment, the machine learning algorithm 20 may even be an intrinsic part of the hearing device circuitry itself. The resulting mean values of the dataset 31 of resulting gain value ranges 30 calculated by the mean value block 28 provided by the system 4 may then be loaded directly into OTC hearing device memory, thus completing a first fitting of the OTC hearing device without the involvement of an HCP. In these embodiments, computational resources may be limited. The trained ensemble 23 of fitting models 22 may thus be implemented as one single DNN 40 which is then loaded with the node weights of each of the particular DNNs N1, N2...Nn of the ensemble 23 from memory in turn when predicting the corresponding dataset 25 of intermediate gain values 26. The hearing device 1 thus emulating the predictive functionality of each DNN 40 in turn and collecting the results as a plurality of datasets 27 as described in the foregoing.

In a fourth use case, the fitting of a hearing device 1 may be checked, perhaps at regular intervals, say, once every three months, using the proposed method. This may be performed by providing the system 4 with e.g., a fresh audiogram 17i and other relevant data in order to assess whether the gain values 18i currently used by the hearing device 1 are still within acceptable limits when checked against the new gain value ranges 30 predicted by the system 4. This could also permit the user to fine-tune the hearing device 1 as the user 2 ages and/or gets more experienced in using the hearing device 1. In embodiments where the system 4 is built into the hearing device 1, for instance, when the user has self-fitted an OTC hearing device, this check may even be performed by the user 2 without any third-party intervention, applying any changes to the current hearing device fitting as needed when the check has been performed. This feature could benefit e.g., patients suffering from Meniere's disease which, among other discomforts, is characterized in a fluctuating hearing loss changing character, maybe several times a day. Being able to perform frequent refitting of a hearing device could potentially contribute to alleviating some of the discomfort experienced by these users. In some embodiments, the hearing device may also perform the listening tests producing the audiogram 17i, e.g., using a BPTA listening test as described in the foregoing. The HCP then only needs to be contacted in cases where a newly predicted fitting exceeds the capabilities of the OTC hearing device, or in other cases where a more elaborate adjustment of the hearing device settings is required.

In some alternative embodiments, the set of input data 12i to the system 4 is extended with e.g., a set of user mood levels detectable by the system 4 or selectable by the user prior to putting on their hearing devices every morning. Based on the user mood level, the system 4 could e.g., predict and enable a fitting with more emphasis on speech reproduction for when the user wants to stay alert and focused, and a calmer, more relaxed fitting for when the user wants to take some time off, but still benefit from a hearing loss compensation.

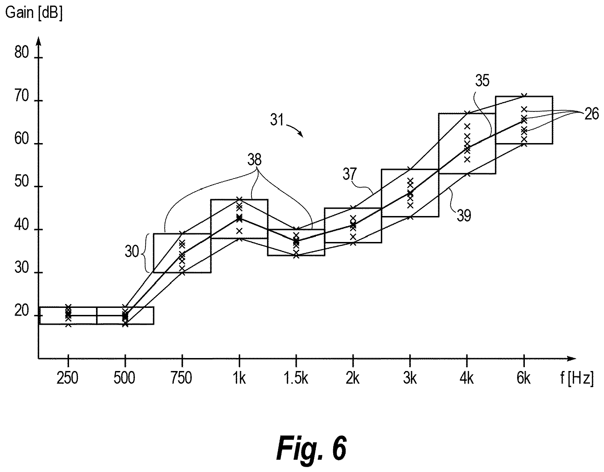

A dataset 31 of resulting gain value ranges 30 provided by the system 4 illustrated in fig. 5 may, for example, be presented in a manner of the diagram illustrated in fig. 6. The diagram shows a dataset 31 of gain ranges 30 for nine different frequency bands; 250 Hz, 500 Hz, 750 Hz, 1 kHz, 1.5 kHz, 2 kHz, 3 kHz, 4 kHz, and 6 kHz. The diagram in fig. 6 is a log-log plot with frequency (in Hertz) indicated on the abscissa and gain values (in Decibels) indicated on the ordinate. The gain value range 30 for each frequency band in fig. 6 is illustrated by a box 38 at the center of each frequency band (for clarity, only the boxes at 750 Hz, 1 kHz, and 1.5 kHz are marked in fig. 6). The height of each box 38 illustrates the upper limit and the lower limit of the gain value range 30 of each frequency band corresponding to the upper gain value and the lower gain value, respectively, for each frequency band as predicted by an ensemble 23 of fitting models 22 of the system 4, and the width of each box 38 illustrates the width of the frequency band (not to scale). The height of each box 38 corresponds to the standard deviation of the intermediate gain values 26 as calculated by the standard deviation block 29 in fig. 5. Between the upper and lower gain limit in each box 38 a number of cross-marks are dispersed, each cross-mark representing an intermediate gain value 26 below the upper gain limit and above the lower gain limit. The set of upper limit gain values for each frequency band is illustrated by an upper line 37, and the set of lower limit gain values for each frequency band is illustrated by a lower line 39. A set of mean gain values is illustrated by a middle line 35 in fig. 6.

The mean gain values represented by the line 35 are calculated by the mean value block 28 in the system 4 as e.g., an arithmetic mean, a median, a quadratic mean, or any other suitable, averaging method as stated in the foregoing. The upper gain values, represented by the line 37, and the lower gain values represented by the line 39, are derived by calculating the standard deviation of the average gain values in the standard deviation block 29 in the system 4. The upper gain values represent the highest gain value predicted by the system 4 for each frequency band, and thus, the highest recommended amplification level to be applied to that frequency band for compensating a given hearing loss. The lower gain values represent the lowest predicted gain value for each frequency band, and thus, the lowest recommended amplification level to be applied to that frequency band for compensating a given hearing loss.

In many cases the recommended amplification level is dependent on the input level. The user profile data 12i may therefore be adapted to accommodate an audiogram 17i taken at e.g., 65 dB and at 85 dB. When the system 4 predicts gain levels for the different frequency bands it will then be capable of predicting two different lower gain levels corresponding to input levels at 65 dB and 85 dB, respectively, for a particular frequency band. In some embodiments, the system 4 may thus provide for e.g., two upper gain value lines 37 and two lower gain value lines 39.

It has been shown that it is possible to use a trained deep neural network to predict a set of fitting parameters in the form of gain value ranges suitable for fitting a hearing aid using the proposed method. The gain values thus obtained by the method may be used as a guideline for an HCP to perform a successful first fitting of a hearing device to a person suffering from a hearing loss, or the gain values may be applied directly to e.g., an OTC hearing aid in a setting where the prospective hearing device user may be guided through a fitting session resulting in a satisfying, first fitting without any aid of the HCP.

Future developments of the method may e.g., include the results of a questionnaire in the user profile data 12i presented to the system 4 prior to predicting a hearing device fitting. The results of such a questionnaire may, for instance, be used to provide each of the fitting models 22 in the ensemble 23 of the machine learning algorithm 20 with a bias, giving the individual fitting models 22 different importance when the averages and the standard deviations are calculated for the intermediate gain values predicted.

Another envisioned development includes the application of a particular sound environment to the ensemble 23 of fitting models 22 for the purpose of using the system 4 to optimize a hearing device fitting for use in that particular sound environment. One way this could be done could be to record a particular sound environment using e.g., a mobile device, sending this recording to a cloud-based service for analysis and training of a corresponding DNN, and then have the cloud-based service send the resulting DNN parameters back to the hearing device for fine-tuning the existing fitting to that particular sound environment. Beneficially, the DNN parameters obtained in this way may subsequently be stored in the hearing device as a user-defined program for future use in similar sound environments.

In some alternative embodiments, the training dataset 24 and the untrained ensemble 23 of fitting models 22 may reside on a remote server. This has two obvious benefits; Firstly, the training dataset 24 may be continuously expanded in order to encompass still more users and fitted hearing devices, even including brand new hearing device models, and the ensemble 23 of fitting models 22 is then trained accordingly in order to acquire the capability to predict correct fittings of new hearing device models almost as soon as they get introduced to the market and a sufficient amount of fitting data for the new models are entered into the training dataset 24. Thus, the ensemble 23 of fitting models 22 is always up to date whenever a new hearing device fitting is performed with the system 4.

Secondly, it may relieve the burden of completing the training procedure itself from a system 4 residing at an HCP or being built into a hearing device by offering the set of DNN node weights of the ensemble 23 of fitting models 22 as a continuously updated database of DNN node weights made available online to an HCP or a hearing device user for download from the remote server to a local system 4 prior to predicting a hearing device fitting, the only prerequisite being that the structure of the ensemble 23 of fitting models 22 residing on the remote server is identical to the ensemble 23 of fitting models 22 residing in the local system 4, either at an HCP or within a hearing device. After downloading a file comprising an updated set of DNN node weights for the ensemble 23 of fitting models 22 from the remote server, the system 4 is ready to predict a hearing device fitting right away using the method. On the remote server, updating training sessions may be performed during idle time, thus always having an updated set of DNN node weights available for download. Apart from the improved convenience for the HCP, this also has the benefit of an increased security against third-party access to the hearing device user data since this data is residing on the remote server and thus not shared. Beneficially, successful hearing device fittings generated by the system 4 may be sent to the remote server and entered into the training dataset 24.

Although the method for fitting a hearing device has been described with reference to a particular implementation, it may be realized in several other ways without deviating significantly from the method and system defined by the claims.

### REFERENCE LIST

- 1: hearing device
- 2: hearing device user
- 4: hearing device fitting system
- 5: processor
- 6: algorithm input
- 7: audiogram datapoint
- 7i: individual audiogram datapoint
- 8: algorithm output
- 10: gain value
- 11: dataset of gain values
- 12: user profile dataset
- 12i: individual user profile dataset
- 13: plurality of user profile datasets
- 14: hearing device dataset
- 14i: particular hearing device dataset
- 15: plurality of hearing device datasets
- 16: user dataset
- 16i: individual user dataset
- 17: audiogram
- 17i: individual audiogram
- 18: corresponding gain value
- 18i: individual gain value
- 19: dataset of corresponding gain values
- 20: machine learning algorithm
- 21: plurality of datasets of corresponding gain values
- 22: fitting model
- 23: ensemble of fitting models
- 24: training dataset
- 25: dataset of intermediate gain values
- 26: intermediate gain value
- 27: plurality of datasets of intermediate gain values
- 28: mean value block for intermediate gain value
- 29: standard deviation block for intermediate gain value
- 30: resulting gain value range
- 31: dataset of resulting gain value ranges
- 32: terminal
- 33: application of individual gain value
- 34: statistical value
- 35: dataset of mean values of intermediate gain values
- 37: dataset of upper values of intermediate gain values
- 39: dataset of lower values of intermediate gain values
- 40: neural net
- 42: input layer
- 44: first hidden layer
- 46: second hidden layer
- 48: output layer
- 50: node weight

## Claims

1. A computer implemented method of determining a gain value range, the method comprising the steps of:
- receiving an input (6) with an individual user hearing characteristic (17i);
- determining a plurality (27) of intermediate gain values (26) using a machine learning algorithm (20) comprising a trained ensemble (23) of fitting models (22) based on the hearing characteristic (17i), wherein at least two fitting models (22) of the trained ensemble (23) of fitting models (22) are each used to determine an intermediate gain value (26);
- determining a statistical value (34) based on the plurality (27) of intermediate gain values (26);
- determining a gain value range (30) corresponding to the individual user hearing characteristic (17i) based on the statistical value (34); and
- outputting the gain value range (30).

2. The method according to claim 1, wherein the input (6) comprises an individual user dataset (16i).

3. The method according to claim 1 or 2, wherein the input (6) comprises a particular hearing device dataset (14i).

4. The method according to any of claims 1-3, wherein the step of determining a statistical value (34) comprises determining a mean value and a standard deviation of the plurality (27) of intermediate gain values (26).

5. The method according to any of the preceding claims, wherein the individual user hearing characteristic (17i) corresponds to a user's hearing ability in a particular frequency band, and wherein the gain value range (30) corresponds to the particular frequency band.

6. The method according to any of the preceding claims, wherein an individual gain value (18i) is determined based on the gain value range (30), and preferably on the mean value of the plurality (27) of intermediate gain values (26).

7. The method according to claim 6, wherein the individual gain value (18i) is applied (33) to a hearing device (1).

8. A computer-based system (4) for determining a gain value range, the system (4) comprising
- means for providing an input (6) with an individual user hearing characteristic (17i);
- a processor (5) configured for executing:
a machine learning algorithm (20) for determining intermediate gain values (26) based on the user hearing characteristic (17i), the machine learning algorithm (20) comprising a trained ensemble (23) of at least two fitting models (22), wherein each fitting model (22) is embodied as a deep, neural network (40);
- a statistical algorithm (28, 29) for determining a statistical value (34) based on a plurality (27) of determined, intermediate gain values (26), and
- a gain value range algorithm for determining a gain value range (30) based on the statistical value (34), and
- means for outputting the determined gain value range (30).

9. The system (4) according to claim 8, wherein the processor (5) is embodied as part of a fitting instrument configured for fitting a hearing device (1).

10. The system (4) according to claim 8, wherein the processor (5) is embodied as part of a general-purpose computer configured for fitting a hearing device (1).

11. The system (4) according to claim 8, wherein the processor (5) is embodied as part of the hearing device (1).

12. The system (4) according to any of claims 8-11, wherein each fitting model (22) of the trained ensemble (23) is embodied as a deep, neural network (40).

13. The system (4) according to any of claims 8-12, wherein the processor (5) is configured to determine an individual gain value (18i) based on the mean value of the plurality (27) of intermediate gain values (26).

14. The system (4) according to any of claims 8-13, wherein the processor (5) is configured to apply (33) the individual gain value (18i) to a hearing device (1).

15. The system (4) according to any of claims 8-14, wherein a set of parameters of the machine learning algorithm (20) is stored at a physical location remote from the system (4), and the set of parameters is retrieved from the remote, physical location by the system (4) and applied to the ensemble (23) of fitting models (22) prior to receiving the individual user hearing characteristic (17i).
